# EUROPEAN PATENT SPECIFICATION

(11) **EP 3 923 893 B1**
(45) Date of publication and mention of the grant of the patent: **14.01.2026**
(21) Application number: 19915027.7
(22) Date of filing: 15.02.2019
(51) Int. Cl.: A61F 13/56, A61F 13/47, A61F 13/511, A61F 13/514

(54) **SANITARY ARTICLE**
HYGIENEARTIKEL
ARTICLE D'HYGIÈNE

(43) Date of publication of application: 22.12.2021
(73) Proprietor: Essity Hygiene and Health Aktiebolag, 405 03 Göteborg (SE)
(72) Inventor: BLOMSTRÖM, Philip, 405 03 GÖTEBORG (SE); SAMUELSSON, Ann, 405 03 Göteborg (SE)
(74) Representative: Hoffmann Eitle
(86) International application number: PCT/SE2019/050132
(87) International publication number: WO 2020/167173

(56) References cited:
- EP-A1- 0 705 585
- EP-A1- 0 923 921
- EP-A2- 1 290 995
- WO-A1-2016/032488
- WO-A1-2017/186935
- GB-A- 2 358 588
- US-A1- 2018 214 318

## Description

### TECHNICAL FIELD

The present invention relates to a sanitary article. The present invention in particular relates to a sanitary article having an improved body fit.

### BACKGROUND OF THE INVENTION

The term "sanitary articles" refers to products that are placed against the skin of the wearer to absorb and contain body exudates, like urine, faeces and menstrual fluid. The disclosure mainly refers to disposable sanitary articles, which means articles that are not intended to be laundered or otherwise restored or reused as a sanitary article. Examples of disposable sanitary articles include feminine hygiene products such as sanitary napkins, panty liners, incontinence pads and the like.

Sanitary articles are generally provided with an absorbent core to receive and retain body liquids. It is also desirable that such sanitary articles are comfortable and have a good fit, i.e. that the sanitary article conforms to the body of the wearer and provides the wearer with a feeling of security and comfort. Additionally, there is desire that such articles are discreet, thin and not visible even if the user wears tighter clothes, which may be seen as being in contradiction with the above objects.

Sanitary articles are generally kept in place in the undergarment of the wearer by means of adhesive provided on a garment-facing side of the sanitary articles. The adhesive provided on the backsheet of sanitary articles prevents the articles from displacement during use.

WO 2016/032488 A1 discloses an absorbent article which can be more readily adaptable to meet the needs of the individual wearer of the absorbent article. The absorbent article includes a topsheet layer, a backsheet layer, and an absorbent core between the topsheet layer and the backsheet layer. The absorbent article further includes an extendable portion capable of being utilized by the wearer to provide additional area of coverage as deemed suitable by the wearer of the absorbent article.

It is an object of the present invention to provide an improved sanitary article which is comfortable to wear and conforms to the body of the user and has an improved fit.

### SUMMARY OF THE INVENTION

One or more of the above objects may be achieved with a sanitary article in accordance with claim 1. Further embodiments are set out in the dependent claims, in the following description and in the drawings.

The sanitary article as disclosed herein has longitudinal side edges extending in a longitudinal direction and transverse front and rear end edges extending in a transverse direction. The sanitary article comprises a fluid permeable surface layer and a backsheet. The backsheet is provided with an adhesive in at least one adhesive region arranged on an outwardly oriented side for attachment of the sanitary article to an undergarment on a user-facing side of said undergarment and at least one adhesive-free region arranged on an outwardly oriented side for attachment of the sanitary article to an undergarment on a user-facing side of said undergarment. The backsheet is divided by an imaginary dividing line in a front portion and a rear portion as seen in the longitudinal direction, the front portion and the rear portion being of equal length and corresponding to portions of the backsheet intended to face the user-facing side of the undergarment during use. The surface layer is an air-through-bonded fibrous nonwoven surface layer having a basis weight of from 14 to 30 g/m² and a density of from 20 to 90 kg/m³. The adhesive-free region extends in the longitudinal direction and the transverse width of the adhesive-free region is from 7% to 40% of the total transverse width(w_{T}) of said backsheet, as measured at a widest transverse width of said backsheet in said rear portion.

The adhesive-free region may be from 10% to 30% of the total transverse width(w_{T}) of said backsheet, as measured at a widest transverse width of said backsheet in said rear portion.

The adhesive free region may also extend in the same longitudinal direction also along the front region. The adhesive free region may extend along the entire length in the longitudinal direction of the backsheet.

The backsheet may be provided with more than one adhesive region arranged on an outwardly oriented side for attachment of the sanitary article to an undergarment on a user-facing side of said undergarment and more than one adhesive-free region arranged on an outwardly oriented side for attachment of the sanitary article to an undergarment on a user-facing side of said undergarment, such as for example 2 to 4 adhesive regions and 2 to 5 adhesive-free regions, wherein each of the adhesive regions and each of the adhesive-free regions extends in the longitudinal direction and the transverse width of at least one adhesive-free region is from 7% to 40% of total transverse width(w_{T}) of said backsheet, as measured at a widest transverse width of said backsheet in said rear portion.

The adhesive-free region being from 7% to 40% of total transverse width(w_{T}) of said backsheet, as measured at a widest transverse width of said backsheet in said rear portion may have a transverse width of at least 8 mm, 10 mm, 12 mm or 15 mm.

The rear portion may be divided, as seen in the transverse direction, in a central rear portion and in first and second lateral rear portions extending from the dividing line and towards the rear end edge. Either, the adhesive region may cover the central rear portion forming a central rear adhesive region and the first and second lateral rear portions may be free from adhesive, or, the central rear portion may be free from adhesive and the adhesive region may cover the first and second lateral rear portions forming first and second lateral rear adhesive regions.

The sanitary article may comprise wings or flaps provided with attachment means, such as adhesive. The first and the second lateral rear end portions are parts of the rear portion and do not include wings or flap as these are not intended to face the user-facing side of the undergarment during use. Hence, the wings or flaps may be provided with adhesive while the first and second lateral rear portions are free from adhesive. Also, when measuring the widest transverse width of said rear portion, the transverse width of said rear portion does not include the wings or flaps.

The term "sanitary articles" refers to products that are placed against the skin of the wearer to absorb and contain body exudates, like urine, faeces and menstrual fluid. The disclosure mainly refers to disposable sanitary articles, which means articles that are not intended to be laundered or otherwise restored or reused as a sanitary article. Examples of disposable sanitary articles include feminine hygiene products such as sanitary napkins, panty liners, incontinence pads and the like.

The air-through-bonded fibrous nonwoven surface layer having a basis weight of from 14 to 30 g/m² and a density of from 20 to 90 kg/m³ has been found to provide the surface layer with surprisingly low friction values both under dry and wet conditions. It was discovered that the low friction surface layer in combination with the adhesive-free region that extends in the longitudinal direction in the rear region and has a transverse width that is at least 7%, or between 7% to 40%, as measured at the widest transverse width of said backsheet in the rear portion promotes the sanitary article to conform with the body to a higher extent as it is not limited by the attachment to the undergarment. Furthermore, the surface layer of air-through bonded nonwoven shows low friction against a user's skin both in dry, moist and wet condition. The surface layer also reduces the risk of liquid flow-off from the wearing facing surface of the topsheet of the sanitary article.

In the air-through bonding process, hot air is passed through the fibrous web to heat and melt polymer fibers. Molten polymer subsequently flows to the point of contact between any two fibers to produce a bond. The fibers in the nonwoven surface layer are thermoplastic polymeric fibers. One reason behind the surprisingly low friction of the material may be that the air-through bonding process enables a reduced number of binding points between the fibers giving the material a very soft and smooth surface. The relatively low number of binding points between the fibers may decrease the friction between the nonwoven and the user skin in presence of moisture/liquid. The low number of binding points also provides the material with a bulky structure facilitating liquid inlet.

If the adhesive region is arranged on the central rear portion, a transverse width of the central rear adhesive region may be from 5% to 70%, optionally from 5% to 40%, or from 10% to 35%, of a total transverse width of the backsheet, as measured at a widest transverse width of the backsheet in the rear portion.

The adhesive-free first and second lateral rear portions may extend from the dividing line and to the rear end edge and/or the central rear adhesive region extends from the dividing line and to the rear end edge. If the adhesive region is arranged on the first and second lateral rear regions, a transverse width of the first and second lateral rear adhesive regions may each be from 5% to 40%, optionally 10% to 40%, or 10% to 35%, as measured at a widest transverse width of the backsheet in the rear portion.

The air-through bonded fibrous nonwoven may comprise synthetic fibers.

The air-through-bonded fibrous nonwoven may comprise bicomponent fibers. At least 80% of the fibers in the air-through-bonded fibrous nonwoven may be bicomponent fibers, such as from 80% to 100% of the fibers may be bicomponent fibers. Optionally all the fibers in the air-through-bonded fibrous nonwoven may be bicomponent fibers.

The bi-component fibers may be sheath-core bicomponent fibers, wherein the sheath may be polyethylene or polyethylene. The core in the sheath-core bicomponent fibers may be of polyester.

The bi-component fibers may be sheath-core bicomponent fibers, wherein the sheath may be of polyethylene and the core may be of polyester. Such fibers have been found to provide resilient structures with low friction and high drapability. A resilient structure gives the air-through-bonded fibrous nonwoven high bulk which improves the liquid absorption into the fabric. The fibers of the air-through-bonded nonwoven may have a coarseness of from 1.8 to 10 dTex,or 2 to 7 dtex.

The outwardly oriented side of the backsheet may comprise a liquid impermeable plastic film. Plastic films have lower friction against undergarment compared to liquid impermeable nonwoven materials permitting the sanitary article to conform with the body to a higher extent and not being limited by friction against the undergarment. The plastic film may be a polyolefin based film. The plastic film may have a basis weight of from 10 to 25 g/m².

The sanitary article may comprise an intermediate layer located between the surface layer and the backsheet, the intermediate layer may be being an airlaid, high-loft nonwoven such as air-through bonded nonwoven material or hydroentangled nonwoven material. The intermediate layer may have a basis weight of from 30 to 120 gsm, or 30 to 80 gsm, or 30 to 70 gsm. The intermediate layer may extend under from 70% to 100% of the surface layer.

The surface layer and the intermediate layer may be adhesively attached to each other. The surface layer and the intermediate layer may also be attached to each other by thermo-and or mechanical welding, such as for example by ultrasonic welding.

The sanitary article may have a first thickness of not more than 8 mm, such as not more than 6 or 4 mm, in a thinner rear portion, the thinner rear portion having a length of about 30% of the total length of the sanitary article, from the rear end edge and towards the front end edge. The thinner rear portion may extend from the first to the second longitudinal edge of the sanitary article.

The fact that the sanitary article may have a thinner rear portion may, in combination with the low-friction surface, promote improved body conformity of the sanitary napkin as the flexibility of a thinner sanitary article generally is improved.

The surface layer may be embossed with an embossing pattern.The embossing pattern may cover from 3% to 20% of the surface layer. An embossed air through bonded fibrous surface layer according to claim 1 has been found to enhance the optical appearance of the embossing pattern by increasing the distinctness of the embossing pattern. One reason may be that such an air through bonded fibrous nonwoven has relatively low number of bonding points between the fibers within the material which enhances the visibility of the embossing pattern when provided on the material.

The sanitary article may have continuous embossed lines such as for example compressions arranged in the longitudinal direction, at least in the rear portion, to further promote body conformity of the sanitary napkin. Embossing lines creating compression lines may extend through the surface layer and also through underlying layer(s) and may also be arranged in other ways in order to further promote body conformity of the sanitary napkin. The sanitary napkin may also have channels, for example in the absorbent core, arranged in different ways, to further promote body conformity of the sanitary napkin.

The surface layer may be free from lotions and/or lubricating agents. As the surface layer in it-self has been found to provide the surface layer with surprisingly low friction values both under dry and wet conditions, lotions and lubricant agents may not be needed to decrease the friction between the nonwoven and the user's skin.

The absorbent article may be a sanitary napkin.

### BRIEF DESCRIPTION OF THE DRAWINGS

The present invention will be further explained hereinafter by means of non-limiting examples and with reference to the appended drawings wherein:
- Fig. 1: shows a top plan view of a sanitary napkin as disclosed herein and as seen from a topsheet side;
- Fig. 2: shows a top plan view of a sanitary napkin as disclosed herein and as seen from the backsheet side;
- Fig. 3: shows a top plan view of an alternative sanitary napkin as disclosed herein and as seen from the backsheet side;
- Fig. 4: shows friction measurement result on surface material.

### DETAILED DESCRIPTION

The invention will be described more closely below by reference to an exemplary embodiment. The invention may however be embodied in many different forms and should not be construed as limited to the embodiments set forth in the drawings and the description thereto.

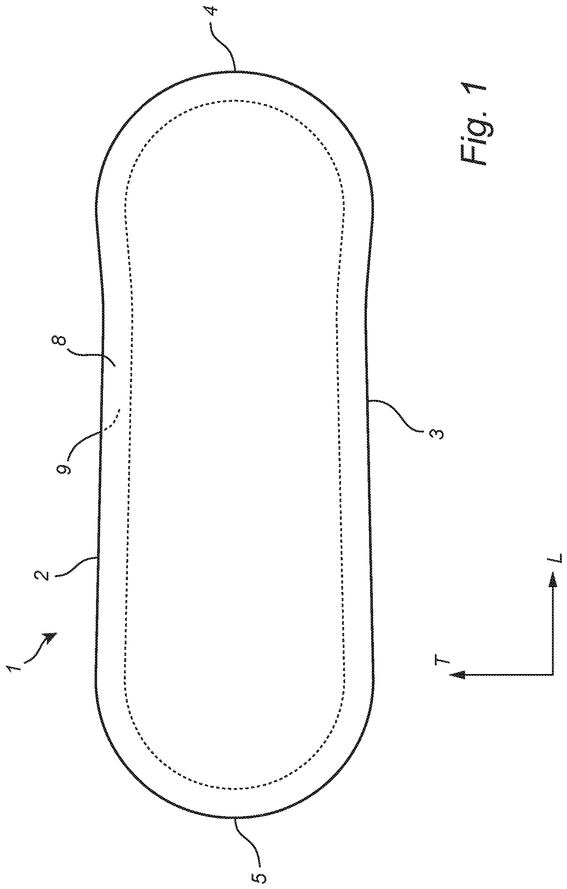

Figure 1 is a top plan view of a sanitary article 1 having longitudinal side edges 2,3 extending in a longitudinal direction L and transverse front and rear end edges 4,5 extending in a transverse direction T. The sanitary article 1 comprising a fluid permeable surface layer 8 and a backsheet 9. The surface layer 8 is an air-through-bonded fibrous nonwoven surface layer having a basis weight of from 14 to 30 g/m² and a density of from 20 to 90 kg/m³. The nonwoven layer comprises bicomponent fibers and may constitute of from 50% or more, such as 80% to 100% or from 95% or more, of bicomponent fibers. The bicomponent fibers may be sheath-core bicomponent fibers, preferably wherein the core is a polyester core and the sheath is a polyethylene sheath. The sanitary napkin 1 may include an absorbent core between the surface layer 8 and the backsheet 9.

The absorbent core may be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent structure. It is also common to have absorbent structures comprising layers of different material with different properties with respect to liquid acquisition capacity, liquid distribution capacity and storage capacity. This is well-known to the person skilled in the art and does therefore not have to be described in detail. The thin absorbent bodies, which are common in today's sanitary articles, often comprise a compressed mixed or layered structure of cellulosic fluff pulp and superabsorbent. The size and absorbent capacity of the absorbent structure may be varied to be suited for different uses such as sanitary articles, pantyliners, adult incontinence pads and diapers, baby diapers, pant diapers, etc.

The sanitary article 1 may be a thin sanitary article 1, having a first thickness of not more than 8 mm in a thinner rear portion, the thinner rear portion having a length of about 30% of the total length of the sanitary article 1.

The backsheet may consist of a thin plastic film, e.g. a polyethylene or polypropylene film, a nonwoven material coated with a liquid impervious material, a hydrophobic nonwoven material, which resists liquid penetration. Laminates of plastic films and nonwoven materials may also be used. The backsheet material may be breathable so as to allow vapor to escape from the absorbent structure, while still preventing liquids from passing through the backsheet material. Preferably the outwardly oriented side of the backsheet is a plastic film. A plastic film provides less friction against undergarment than a fibrous nonwoven material and thus promotes that the sanitary article conforms well to the body of the wearer.

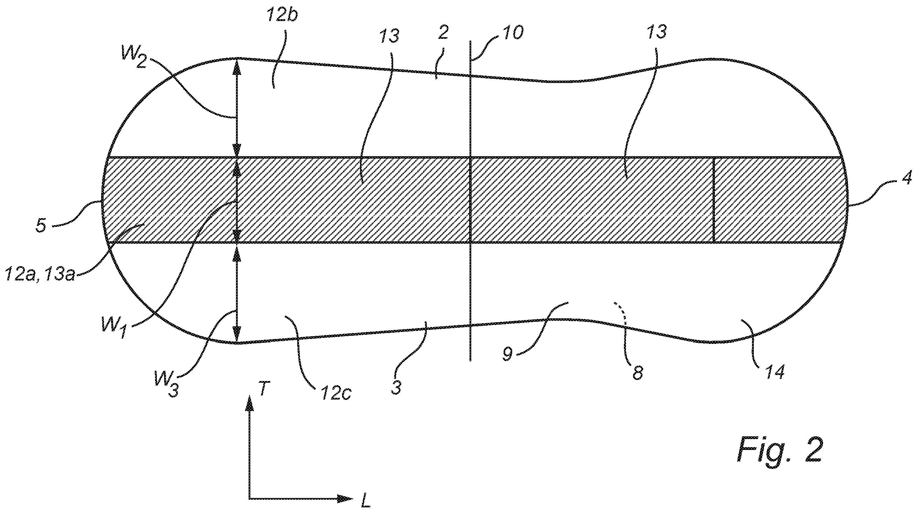

Fig. 2 is a top plan view of the sanitary article 1, as seen from the backsheet 9, being the side intended to face a user-facing side of an undergarment during use. The backsheet 9 is divided by an imaginary dividing line 10 in a front portion and a rear portion as seen in said longitudinal direction L. The front portion and the rear portion are of equal length. The rear portion is divided, as seen in the transverse direction T, in a central rear portion 12a and in first and second lateral rear portions 12b, 12c extending from the dividing line 10 and towards the rear end edge 5. The backsheet 9 is provided with an adhesive in an adhesive region 13 arranged on an outwardly oriented side 14 thereof. The adhesive region 13 covers the central rear portion 12a forming a central rear adhesive region 13a and the first and second lateral rear portions 12b, 12c are free from adhesive. The adhesive is an adhesive conventionally used on backsheets for sanitary napkins for securing the sanitary napkin to the undergarment of the user. A transverse width w₁ of the central rear adhesive region 13a is from 10% to 70%, optionally from 10% to 40%, of a total transverse width w_{T}, i.e. w₁+w₂+w₃, of the backsheet 9, as measured at a widest transverse width of said backsheet 9 in said rear portion.

The adhesive-free first and second lateral rear portions 12b, 12c and the central rear adhesive region 13a each extends from the dividing line 10 and to the rear end edge 5. However, the central rear adhesive region 13a may also extend close to the rear end edge 5, such as for example up to 10 mm from the rear end edge 5.

The front portion 14 is provided with adhesive in the adhesive region arranged in a central front region. However, the front region may have an adhesive region extending over the entire regions or only parts thereof.

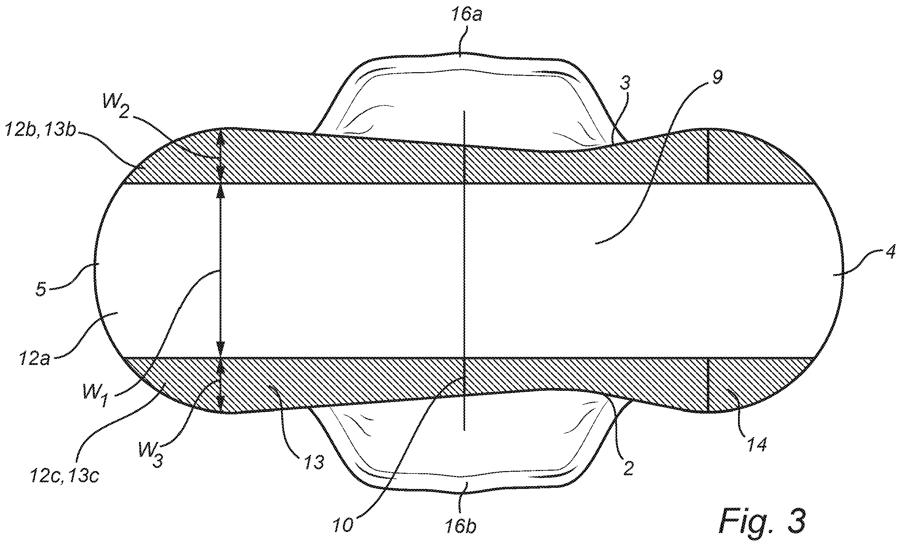

Fig. 3 is a top plan view of an alternative sanitary napkin 1 according to the present disclosure. The sanitary napkin 1 is shown from a backsheet 9 side. The backsheet 9 is divided by an imaginary dividing line 10 in a front portion and a rear portion as seen in said longitudinal direction L. The front portion and the rear portion are of equal length. The rear portion is divided, as seen in the transverse direction T, in a central rear portion 12a and in first and second lateral rear portions 12b, 12c extending from the dividing line 10 and towards the rear end edge 5. The backsheet 9 is provided with an adhesive in an adhesive region 13 arranged on an outwardly oriented side 14 thereof. The adhesive region 13 covers the first lateral rear portion 12b and the second lateral rear portion 2c, thereby forming a first lateral rear adhesive region 13b and second lateral rear adhesive region 13c. The central rear portion 12a is free from adhesive. The adhesive is an adhesive conventionally used on backsheets for sanitary napkins for securing the sanitary napkin to the undergarment of the user. A transverse width of each of the first and second lateral rear adhesive regions 13b, 13c may be from 5% to 40%, as measured at a widest transverse width of the backsheet 9 in the rear portion. The transverse width of the backsheet is measured on the part of the sanitary napkin 1 facing the wearer facing side of an undergarment. Thus, for a sanitary napkin provided with lateral wings, these are not to be included for such measurements.

The sanitary napkin is provided with a pair of lateral wings 16a,16b extending outward from the transversely opposite side edges 2,3 of the napkin. The wings are provided with attachment means, such as with an adhesive, on their garment facing surface so that the wings 16a, 16b can be folded back under the undergarment and attached to the garment facing side of the undergarment. In this way, the wings 16a, 16b serve to keep the napkin 1 properly positioned in the undergarment.

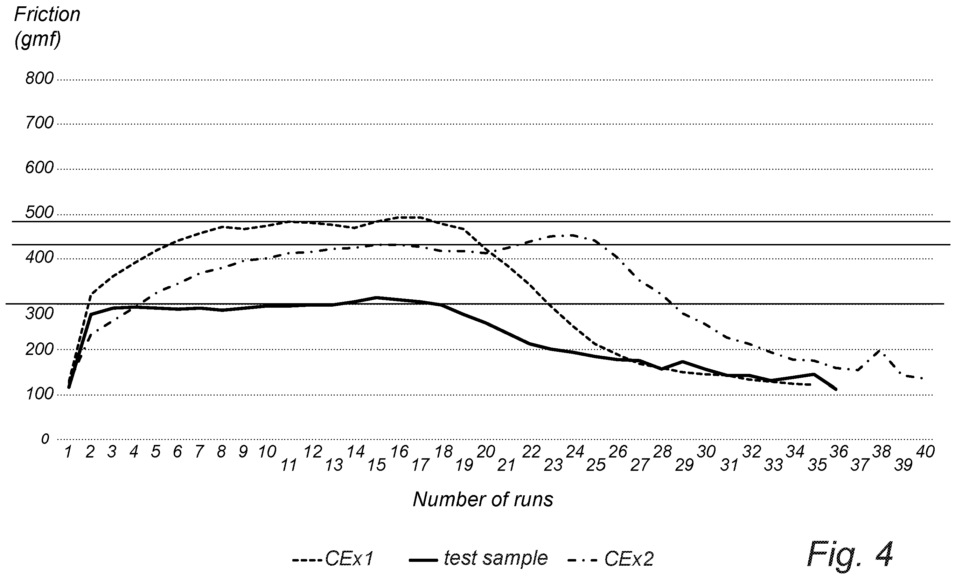

Figure 4 shows three friction curves, the test sample according to the present invention, and two comparative examples, CEx 1 and CEx 2. These samples are described more in detail in table 1. In figure 4 is the number of runs on the x-axis and the friction force in gmf on the y-axis. In figure 4 is the number of runs on the x-axis and the friction force in gmf on the y-axis. A friction curve comprises a first slope having a positive coefficient illustrating increase in the friction values, a plateau, and a second slope having a negative coefficient illustrating decrease in friction values. At the plateau, the friction values are substantially constant over the extension of the plateau. Small variations at the plateau as well as along the slopes are possible between individual values, but with a positive coefficient is meant that all individual values in the first slope together creates a positive coefficient, as well as all individual values in the second slope together creates a negative coefficient, as well as all individual values in the plateau together creates a plateau. Lower friction values render the absorbent article more skin friendly and skin problems arising with the use of the absorbent article can be reduced. For some materials a clear peak can be seen in a curve of friction values before the second slope creating a negative coefficient. Such a peak is caused by clinging, which may occur when only a small amount of moisture is present. The result shows that the test sample (air-through-bonded nonwoven surface material) has a lower mean friction plateau value (gmf) than the two comparative examples CEx 1 and CEx 2.

### Friction measurement- Stick and slip method for measuring the wet friction

Friction occurring between a nonwoven material and the skin of the user is different in the presence of liquid/moisture than when no liquid/moisture is present. Even a very small amount of moisture present originating from perspiration, sweat or other body fluids has an impact on the friction forces occurred between the nonwoven material and the skin of the user. It has therefore been discovered that it is really important to carefully choose the nonwoven characteristics, so that the nonwoven is able to minimize the mechanical discomfort during the overall use of the product.

The method used is called Stick and slip measurement method for measuring the wet friction and the method measures the static friction, sns value (stick and slip value) in gram force, gmf, between a material and the human skin. Repeatedly runs are made using the same material strip. First the sns value for the dry state (dry material and skin) is measured followed by wet state at different liquid levels (from completely wetted material, to moist and to almost dry) until the sns value is back to the skin-material interaction level measured in the first dry run, which mean that the material is dry again. The method is thus called a repeated stick and slip method or sns dry-wet-dry. The stick and slip value is defined as the point on the force curve (gmf) where the material starts gliding over the arm. The sns values from all single force curves are then put together in a new graph, sns values as a function of number of runs.

Three different nonwoven materials were tested and compared in terms of dry friction and wet friction. The test material is an air-through-bonded nonwoven according to the present disclosure comprising bicomponent fibers of core-sheath type with a polyester core and a polyethylene sheath. The first Comparative Example is a spunbond nonwoven with polypropylene fibers and the second Comparative Example is spunbond nonwoven with polypropylene fibers. Table 1 below provides specifications of the materials tested.

**Table 1**

| | Material Type | Supplier | Material no | Basis weight (gsm) |
|---|---|---|---|---|
| Test sample | Air-though bonded nw | TWE | 255272 | 20 |
| CEx 1 | Spunbond nonwoven | Texbond | 2436701 | 18 |
| CEx 2 | Spunbond nonwoven | Union | 272119 | 18 |

In Table 2 below shows result of the mean friction plateau values measured in gmf. By gmf is meant gram-force and one gram-force is 9.80665 mN and the result shows that the test sample, the air-through-bonded nonwoven surface material has a lower mean friction plateau value (gmf) than CEx 1 and CEx 2. Also in Figure 4 shows the friction curves for test sample, CEx 1 and CEx2. The air through bonded nonwoven has a lower friction than the spunbonded nonwoven materials.

**Table 2**

| Material | Mean friction plateau value, (gmf) |
|---|---|
| Test sample | 300 |
| CEx 1 | 480 |
| CEx 2 | 420 |

### Initial spreading measurement

The initial spreading for a surface layer material according to the present disclosure was compared with a conventional spunbond surface layer 20 g/m². The method used was the FLOW method and a sanitary napkin according to the present invention and comprising an air through bonded nonwoven 20 g/m² from TWE, as surface layer and combined with an intermediate layer in the form of an airlaid nonwoven material having a basis weight of 65-70 g/m², was compared with a sanitary napkin comprising a surface layer of a spunbonded nonwoven material combined with an intermediate layer in the form of an airlaid nonwoven material 65-70 g/m². The test articles included also a pulp core layer and a liquid impermeable polyolefin plastic film backsheet. Each of the test articles included exactly the same layers except for the surface layer.

The test samples are conditioned in 24 ± 4h at 50 ± 10% r.h. and 23 ± 1 °C. The number of samples for each test type is 6. The samples are placed on a Plexiglas table with an angle of 25°.

According to the FLOW method, 5 ml of test liquid, synthetic menstrual fluid, is added to the sanitary napkins provided with the respective materials as surface layers, the test liquid is added in a dose 1, 2 and 3 with an interval of 15 seconds and with a flow speed of 20ml/min. After this the spreading length (mm) of the liquid was measured for each of the materials and is shown in the table 3 below. When the fluid has been added, the longest distance the fluid has run on top of the surface material is measured.

**Table 3**

| | Test sample (air-through-bonded nonwoven material) | CEx 2 (spunbond nonwoven material) |
|---|---|---|
| Dose 1 | 51 mm | 71 mm |
| Dose 2 | 74 mm | 88 mm |
| Dose 3 | 94 mm | 113 mm |

As illustrated in table 3, the spreading length of the liquid and thereby the wetted surface area is lower for the air-through-bonded nonwoven material. Details regarding the surface fibrous nonwoven surface layers can be seen in Table 1.

### Density measurement

The density is calculated by dividing the basis weight of the fibrous nonwoven surface layer by its thickness measured at a pressure of 0.5 kPa. The thickness is determined by means of a measuring foot with affixed load of 0.5 k Pa having a foot area of 50x50 mm². The thickness is read off at the digital thickness gauge/tester after 10 seconds when the measuring foot has touched the surface of the sample.

### Embossing measurement

The depth of the individual embossed elements in the form of dots have been measured by the method ISO25178 and also the depth of a continuous embossed line extending along a contour of the absorbent sanitary napkin framing the embossed pattern comprising the individual embossed elements have been measured by the method ISO25178. Three different sanitary napkins were tested having different surface layers but otherwise constructed with the same underlying materials and compared in terms of diversity of embossment depth. The test material is an air-through-bonded nonwoven according to the present disclosure comprising bicomponent fibers of core-sheath type with a polyester core and a polyethylene sheath. The first Comparative Example is a spunbond nonwoven with polypropylene fibers and the second Comparative Example is spunbond nonwoven with polypropylene fibers (for details about the material see Table 1). In Table 4 shows the result of the mean individual depth of the individual embossed elements (µm) and the standard deviation of the depth (µm).

**Table 4**

| | Mean individual depth (µm) | Standard deviation (µm) |
|---|---|---|
| Test sample (air-through bonded nonwoven) | 388 | 5.9 |
| CEx 1 (spunbond nonwoven) | 446 | 26.0 |
| Cex 2 (spunbond nonwoven) | 368 | 22.3 |

The result shows that the standard deviation of the depth value of the embossment element for the air-through-bonded nonwoven was lower than the standard depth deviation for the comparative examples, Cex 1 and Cex 2.

**Table 5 - The depth of a continuous embossed line, a "valley"**

| The depth of a continuous embossed line has also been measured by the method ISO25178. | | | |
|---|---|---|---|
| | Mean individual depth (µm) | Standard deviation (µm) | Ratio between Standard deviation and Mean individual depth |
| Test sample | 971 | 148 | 0.15 |
| CEx 1 | 597 | 204 | 0.34 |
| Cex 2 | 442 | 91 | 0.21 |

The mean individual depth of a continuous embossed line, a "valley", for the air-through-bonded nonwoven surface layer according to the Test sample, has a lower ratio between Standard deviation and Mean individual depth than the Comparative example 1 (Cex 1) and the Comparative example 2 (Cex 2). So, both the standard deviation of the mean depth of the individual embossed elements in the form of dots, and the ratio between Standard deviation and Mean individual depth of a continuous embossed line shows that the air-through-bonded nonwoven surface material resulting in more distinct embossed elements that enhances the visibility of the embossing pattern when provided on the material.

## Claims

1. A sanitary article (1) having longitudinal side edges (2,3) extending in a longitudinal direction (L) and transverse front and rear end edges (4,5) extending in a transverse direction (T), said sanitary article (1) comprises a fluid permeable surface layer (8) and a backsheet (9), said backsheet (9) being provided with an adhesive in at least one adhesive region arranged on an outwardly oriented side for attachment of the sanitary article to an undergarment on a user-facing side of said undergarment and at least one adhesive-free region arranged on an outwardly oriented side for attachment of the sanitary article to an undergarment on a user-facing side of said undergarment, said backsheet (9) being divided by an imaginary dividing line (10) in a front portion and a rear portion as seen in the longitudinal direction (L), said front portion and said rear portion being of equal length and corresponding to portions of the backsheet (9) intended to face the user-facing side of the undergarment during use **characterized in that** said surface layer (8) is an air-through-bonded fibrous nonwoven surface layer having a basis weight of from 14 to 30 g/m² and a density of from 20 to 90 kg/m³ and that the adhesive-free region extends in the longitudinal direction and that the transverse width of the adhesive-free region is from 7% to 40% of total transverse width(w_{T}) of said backsheet (9), as measured at a widest transverse width of said backsheet (9) in said rear portion.

2. A sanitary article (1) according to claim 1, wherein the adhesive-free region is from 10% to 30% of the total transverse width(w_{T}) of said backsheet (9), as measured at a widest transverse width of said backsheet (9) in said rear portion.

3. A sanitary article (1) according to claim 1 or 2 wherein the backsheet (9) is provided with more than one adhesive region and more than one adhesive-free region, wherein each of the adhesive regions and each of the adhesive-free regions extends in the longitudinal direction (L) and the transverse width of at least one adhesive-free region is from 7% to 40% of total transverse width(w_{T}) of said backsheet (9) as measured at a widest transverse width of said backsheet (9) in said rear portion.

4. The sanitary article (1) according to claim 1 or 2, wherein said rear portion being divided, as seen in said transverse direction (T), in a central rear portion (12a) and in first and second lateral rear portions (12b,12c) extending from said dividing line (10) and towards said rear end edge (5) and in that said adhesive region (13) cover said central rear portion (12a) forming a central rear adhesive region (13a) and in that said first and second lateral rear portions (12b,12c) are free from adhesive.

5. The sanitary article (1) according to preceding claim, wherein a transverse width (w₁) of said central rear adhesive region (13a) is from 10% to 70%, optionally from 10% to 40%, of a total transverse width(w_{T}) of said backsheet (9), as measured at a widest transverse width of said backsheet (9) in said rear portion.

6. The sanitary article (1) according to claim 4 or 5, wherein said adhesive-free first and second lateral rear portions (12b,12c) extend from said dividing line (10) and to said rear end edge (5) and/or said central rear adhesive region (13a) extends from said dividing line (10) and to said rear end edge (5).

7. The sanitary article (1) according to claim 1 or 2 wherein said rear portion being divided, as seen in said transverse direction (T), in a central rear portion (12a) and in first and second lateral rear portions (12b,12c) extending from said dividing line (10) and towards said rear end edge (5) and in that said central rear portion (12a) is free from adhesive and that said adhesive region (13) cover said first and second lateral rear portions (12b,12c) forming first and second lateral rear adhesive regions (13b,13c).

8. The sanitary article (1) according to claim 7, wherein a transverse width (w₂,w₃) of said first and second lateral rear adhesive regions (13b,13c) each is from 5% to 40%, as measured at a widest transverse width of said backsheet (9) in said rear portion.

9. The sanitary article (1) according to any one of the preceding claims, wherein the air-through-bonded fibrous nonwoven surface layer comprises bicomponent fibers.

10. The sanitary article (1) according to claim 9, wherein said bi-component fibers are sheath-core bicomponent fibers, preferably wherein said core is a polyester core and said sheath is a polyethylene sheath.

11. The sanitary article (1) according to any one of the preceding claims, wherein said fibers of said air-through-bonded nonwoven has a coarseness of from 1.8 to 10 dTex.

12. The sanitary article (1) according to any one of the preceding claims, wherein said outwardly oriented side (14) of said backsheet (9) comprises a liquid impermeable plastic film.

13. The sanitary article (1) according to any one of the preceding claims, wherein said sanitary article (1) comprises an intermediate layer located between said surface layer (8) and said backsheet (9), said intermediate layer comprising a nonwoven material.

14. The sanitary article (1) according to preceding claim, wherein said intermediate layer covers from 70% to 100% of said surface layer (8).

15. The sanitary article (1) according to any one of the preceding claims, wherein at least said surface layer (8) is embossed with an embossing pattern, said embossing pattern covering from 3% to 20% of the surface layer (8).

16. The sanitary article (1) according to any one of the preceding claims, wherein the sanitary article is a sanitary napkin.

## Patentansprüche

1. Hygieneartikel (1), aufweisend Längsseitenränder (2, 3), die sich in einer Längsrichtung (L) erstrecken, und quer verlaufende vordere und hintere Endränder (4, 5), die sich in einer Querrichtung (T) erstrecken, wobei der Hygieneartikel (1) eine flüssigkeitsdurchlässige Oberflächenschicht (8) und eine Rückseitenlage (9) umfasst,
wobei die Rückseitenlage (9) auf einer nach außen gerichteten Seite mit einem Klebstoff in mindestens einem Klebstoffbereich bereitgestellt ist, der zur Befestigung des Hygieneartikels an einem Unterkleidungsstück an einer dem Benutzer zugewandten Seite des Unterkleidungsstücks angeordnet ist und mindestens einen klebstofffreien Bereich aufweist, der auf einer nach außen gerichteten Seite zur Befestigung des Hygieneartikels an einem Unterkleidungsstück an einer dem Benutzer zugewandten Seite des Unterkleidungsstücks angeordnet ist, wobei die Rückseitenlage (9) durch eine imaginäre Trennlinie (10) in einen vorderen Abschnitt und einen hinteren Abschnitt in der Längsrichtung (L) gesehen unterteilt ist,
wobei der vordere Abschnitt und der hintere Abschnitt gleich lang sind und Abschnitten der Rückseitenlage (9) entsprechen, die während der Verwendung der dem Benutzer zugewandten Seite des Unterkleidungsstücks zugewandt sein sollen, **dadurch gekennzeichnet, dass** die Oberflächenschicht (8) eine heißluftverfestigte faserige Vliesoberflächenschicht ist, aufweisend eine Flächenmasse von von 14 bis 30 g/m² und eine Dichte von von 20 bis 90 kg/m³ und dass sich der klebstofffreie Bereich in der Längsrichtung erstreckt und dass die Querbreite des klebstofffreien Bereichs von 7 % bis 40 % der gesamten Querbreite(w_{T}) der Rückseitenlage (9) beträgt, gemessen an einer größten Querbreite der Rückseitenlage (9) in dem hinteren Abschnitt.

2. Hygieneartikel (1) nach Anspruch 1, wobei der klebstofffreie Bereich von 10 % bis 30 % der gesamten Querbreite(w_{T}) der Rückseitenlage (9) beträgt, gemessen an einer größten Querbreite der Rückseitenlage (9) in dem hinteren Abschnitt.

3. Hygieneartikel (1) nach Anspruch 1 oder 2, wobei die Rückseitenlage (9) bereitgestellt ist mit mehr als einem Klebstoffbereich und mehr als einem klebstofffreien Bereich,
wobei sich jeder der Klebstoffbereiche und jeder der klebstofffreien Bereiche in der Längsrichtung (L) erstreckt und die Querbreite von mindestens einem klebstofffreien Bereich von 7 % bis 40 % der gesamten Querbreite(w_{T}) der Rückseitenlage (9) beträgt, gemessen an einer größten Querbreite der Rückseitenlage (9) in dem hinteren Abschnitt.

4. Hygieneartikel (1) nach Anspruch 1 oder 2, wobei der hintere Abschnitt, in der Querrichtung (T) gesehen, in einen mittleren hinteren Abschnitt (12a) und in erste und zweite laterale hintere Abschnitte (12b, 12c) unterteilt ist, die sich von der Trennlinie (10) in Richtung zum hinteren Endrand (5) erstrecken und dadurch, dass der Klebstoffbereich (13) den mittleren hinteren Abschnitt (12a) bedecken, wodurch ein mittlerer hinterer Klebstoffbereich (13a) gebildet wird, und dadurch, dass die ersten und zweiten lateralen hinteren Abschnitte (12b, 12c) frei von Klebstoff sind.

5. Hygieneartikel (1) nach vorstehendem Anspruch, wobei eine Querbreite (w₁) des mittleren hinteren Klebstoffbereichs (13a) von 10 % bis 70 %, wahlweise von 10 % bis 40 %, einer gesamten Querbreite(w_{T}) der Rückseitenlage (9) beträgt, gemessen an einer größten Querbreite der Rückseitenlage (9) in dem hinteren Abschnitt.

6. Hygieneartikel (1) nach Anspruch 4 oder 5, wobei sich die klebstofffreien ersten und zweiten lateralen hinteren Abschnitte (12b, 12c) von der Trennlinie (10) bis zum hinteren Endrand (5) erstrecken und/oder sich der mittlere hintere Klebstoffbereich (13a) von der Trennlinie (10) bis zum hinteren Endrand (5) erstreckt.

7. Hygieneartikel (1) nach Anspruch 1 oder 2, wobei der hintere Abschnitt, in der Querrichtung (T) gesehen, in einen mittleren hinteren Abschnitt (12a) und in erste und zweite laterale hintere Abschnitte (12b, 12c) unterteilt ist, die sich von der Trennlinie (10) in Richtung zum hinteren Endrand (5) erstrecken und dadurch, dass der mittlere hintere Abschnitt (12a) frei von Klebstoff ist und dass der Klebstoffbereich (13) die ersten und zweiten lateralen hinteren Abschnitte (12b, 12c) bedecken, wodurch erste und zweite laterale hintere Klebstoffbereiche (13b, 13c) gebildet werden.

8. Hygieneartikel (1) nach Anspruch 7, wobei eine Querbreite (w₂, w₃) der ersten und zweiten lateralen hinteren Klebstoffbereiche (13b, 13c) jeweils von 5 % bis 40 % beträgt, gemessen an einer größten Querbreite der Rückseitenlage (9) in dem hinteren Abschnitt.

9. Hygieneartikel (1) nach einem der vorstehenden Ansprüche, wobei die heißluftverfestigte faserige Vliesoberflächenschicht Bikomponentenfasern umfasst.

10. Hygieneartikel (1) nach Anspruch 9, wobei es sich bei den Bikomponentenfasern um Kern-Mantel-Bikomponentenfasern handelt, bevorzugt wobei der Kern ein Polyesterkern ist und der Mantel ein Polyethylenmantel ist.

11. Hygieneartikel (1) nach einem der vorstehenden Ansprüche, wobei die Fasern des heißluftverfestigten Vlieses eine Grobheit von von 1,8 bis 10 dTex aufweist.

12. Hygieneartikel (1) nach einem der vorstehenden Ansprüche, wobei die nach außen gerichtete Seite (14) der Rückseitenlage (9) eine flüssigkeitsundurchlässige Kunststofffolie umfasst.

13. Hygieneartikel (1) nach einem der vorstehenden Ansprüche, wobei der Hygieneartikel (1) eine Zwischenlage umfasst, die zwischen der Oberflächenschicht (8) und der Rückseitenlage (9) angeordnet ist, wobei die Zwischenlage ein Vliesmaterial umfasst.

14. Hygieneartikel (1) nach vorstehendem Anspruch, wobei die Zwischenlage von 70 % bis 100 % der Oberflächenschicht (8) bedeckt.

15. Hygieneartikel (1) nach einem der vorstehenden Ansprüche, wobei zumindest die Oberflächenschicht (8) mit einem Prägemuster geprägt ist, wobei das Prägemuster von 3 % bis 20 % der Oberflächenschicht (8) bedeckt.

16. Hygieneartikel (1) nach einem der vorstehenden Ansprüche, wobei der Hygieneartikel eine Damenbinde ist.

## Revendications

1. Un article sanitaire (1) ayant des bords latéraux longitudinaux (2, 3) s'étendant dans une direction longitudinale (L) et des bords d'extrémité avant et arrière (4, 5) transversaux s'étendant dans une direction transversale (T), ledit article sanitaire (1) comprend une couche de surface (8) perméable aux fluides et une feuille arrière (9), ladite feuille arrière (9) étant munie d'un adhésif dans au moins une région adhésive disposée sur un côté orienté vers l'extérieur pour fixer l'article sanitaire sur un sous-vêtement sur un côté faisant face à l'utilisateur de ce sous-vêtement et au moins une région sans adhésif disposée sur un côté orienté vers l'extérieur pour fixer l'article sanitaire à un sous-vêtement sur un côté faisant face à l'utilisateur de ce sous-vêtement, la feuille arrière (9) étant divisée par une ligne de division (10) imaginaire en une partie avant et une partie arrière comme visible dans la direction longitudinale (L), ladite partie avant et ladite partie arrière étant de même longueur et correspondant aux parties de la feuille arrière (9) destinées à faire face au côté faisant face à utilisateur du sous-vêtement lors de l'utilisation **caractérisé en ce que** ladite couche de surface (8) est une couche de surface non tissée fibreuse liée par soufflage d'air, ayant un poids de base de 14 à 30 g/m² et une densité de 20 à 90 kg/m³, et **en ce que** la région sans adhésif s'étend dans la direction longitudinale et **en ce que** la largeur transversale de la région sans adhésif est de 7 % à 40 % de la largeur transversale totale (w_{T}) de ladite feuille arrière (9), telle que mesurée à la largeur transversale la plus large de ladite feuille arrière (9) dans ladite partie arrière.

2. Un article sanitaire (1) selon la revendication 1, où la région sans adhésif est de 10 % à 30 % de la largeur transversale totale (w_{T}) de ladite feuille arrière (9), telle que mesurée à la largeur transversale la plus large de ladite feuille arrière (9) dans ladite partie arrière.

3. Un article sanitaire (1) selon la revendication 1 ou 2 où la feuille arrière (9) est munie de plus d'une région adhésive et de plus d'une région sans adhésif, où chacune des régions adhésives et chacune des régions sans adhésif s'étend dans la direction longitudinale (L) et la largeur transversale d'au moins une région sans adhésif est de 7 % à 40 % de la largeur transversale totale (w_{T}) de ladite feuille arrière (9) telle que mesurée à la largeur transversale la plus large de ladite feuille arrière (9) dans ladite partie arrière.

4. L'article sanitaire (1) selon la revendication 1 ou 2, dans lequel ladite partie arrière est divisée, comme visible dans ladite direction transversale (T), en une partie centrale arrière (12a) et dans les première et deuxième parties latérales arrières (12b, 12c) s'étendant de ladite ligne de division (10) et vers ledit bord d'extrémité arrière (5) et en ce que ladite région adhésive (13) recouvre ladite partie centrale arrière (12a) formant une région adhésive centrale arrière (13a) et en ce que lesdites premières et secondes parties latérales arrières (12b, 12c) sont sans adhésif.

5. L'article sanitaire (1) selon la revendication précédente, selon lequel une largeur transversale (w₁) de ladite région adhésive centrale arrière (13a) est de 10 % à 70 %, éventuellement de 10 % à 40 %, d'une largeur transversale totale (w_{T}) de ladite feuille arrière (9), telle que mesurée à la largeur transversale la plus large de ladite feuille arrière (9) dans ladite partie arrière.

6. L'article sanitaire (1) selon la revendication 4 ou 5, dans lequel les première et seconde parties latérales arrières (12b, 12c) sans adhésif s'étendent de ladite ligne de division (10) jusqu'audit bord d'extrémité arrière (5) et/ou la région adhésive centrale arrière (13a) s'étend depuis ladite ligne de division (10) et jusqu'au bord d'extrémité arrière (5).

7. L'article sanitaire (1) selon la revendication 1 ou 2, dans lequel ladite partie arrière est divisée, comme visible dans ladite direction transversale (T), en une partie centrale arrière (12a) et en des première et deuxième parties latérales arrières (12b, 12c) s'étendant depuis ladite ligne de division (10) et vers ledit bord d'extrémité arrière (5) et en ce que ladite partie centrale arrière (12a) est sans adhésif et que ladite région adhésive (13) recouvre lesdites première et seconde parties latérales arrières (12b, 12c) formant des première et deuxième régions adhésives latérales arrières (13b, 13c).

8. L'article sanitaire (1) selon la revendication 7, selon lequel une largeur transversale (w₂, w₃) desdites première et deuxième régions adhésives latérales arrières (13b, 13c) est chacune de 5 % à 40 %, telle que mesurée à la largeur transversale la plus large de ladite feuille arrière (9) dans ladite partie arrière.

9. L'article sanitaire (1) selon l'une des revendications précédentes, dans lequel la couche de surface non tissée fibreuse liée par soufflage d'air est composée de fibres bicomposantes.

10. L'article sanitaire (1) selon la revendication 9, dans lequel les fibres bicomposantes sont des fibres bicomposantes noyau-gaine, de préférence où ledit noyau est un noyau en polyester et ladite gaine est une gaine en polyéthylène.

11. L'article sanitaire (1) selon l'une des revendications précédentes, dans lequel les fibres dudit non tissé lié par soufflage d'air ont une grossièreté de 1,8 à 10 dTex.

12. L'article sanitaire (1) selon l'une des revendications précédentes, dans lequel le côté orienté vers l'extérieur (14) de ladite feuille arrière (9) comprend un film plastique imperméable aux liquides.

13. L'article sanitaire (1) selon l'une des revendications précédentes, dans lequel ledit article sanitaire (1) comprend une couche intermédiaire située entre ladite couche de surface (8) et ladite feuille arrière (9), ladite couche intermédiaire comprenant un matériau non tissé.

14. L'article sanitaire (1) selon la revendication précédente, dans lequel ladite couche intermédiaire couvre de 70 % à 100 % de ladite couche de surface (8).

15. L'article sanitaire (1) selon l'une des revendications précédentes, dans lequel au moins ladite couche de surface (8) est embossée d'un motif de bossage, ledit motif de bossage couvrant de 3 % à 20 % de la couche de surface (8).

16. L'article sanitaire (1) selon l'une des revendications précédentes, dans lequel l'article sanitaire est une serviette hygiénique.
